# EUROPEAN PATENT APPLICATION

(11) **EP 0 597 699 A2**
(43) Date of publication of application: **18.05.1994**
(21) Application number: 93308993.0
(22) Date of filing: 10.11.1993
(51) Int. Cl.: C12N 9/64, G01N 33/53, G01N 33/68, C12Q 1/68

(54) **Protein secreted by cancer cells**

(30) Priority: 10.11.1992 JP 299680/92
(71) Applicant: TOSOH CORPORATION, Yamaguchi-ken 746 (JP)
(72) Inventor: Hori, Hitoshi, Fujisawa-shi, Kanagawa-ken (JP); Matsuba, Takao, Atsugi-shi, Kanagawa-ken (JP); Yamada, Masayuki, Sagamihara-shi, Kanagawa-ken (JP)
(74) Representative: Kearney, Kevin David Nicholas

(57) **Abstract**

A protein secreted by cancer cells, which has the following characteristics a) it is reactive with an anti-cathepsin E antibody, b) its molecular weight is about 60,000 in the form of a monomer and about 120,000 in the form of a dimer, c) it is secreted from pancreatic cancer cells, and d) it has a protease activity.

## Description

The present invention relates to a protein which is secreted from cancer cells and which is reactive with an anti-cathepsin E antibody.

Among physiologically active substances numerously present in a vital body, there are some which show certain interrelations between their contents and diseases. Namely, by measuring the amount of such a physiologically active substance present in a vital body, it may be possible to detect the disease at an early stage.

Cathepsin E is an enzyme isolated and purified in the form of a dimer having a molecular weight of about 80,000 (Takeda M et al J. Biochem. 100, p1269, 1986). It is localized in the cells constituting an organ such as gastric mucosal epithelium, and it is a protease which may be classified in the category of acidic proteases. Its physiological activities have not yet been fully understood, and its significance has not yet been established among various views. However, from the localization pattern specific to a certain organ, an attention has been drawn to cathepsin E as a possible marker for inflammation or cancer in an organ such as a stomach in which its localization is known. Namely, it has been sought to detect cathepsin E leaked out from cells having a certain trouble and to utilize such detection for diagnosis of e.g. gastritis or stomach cancer at an early stage.

For a physiologically active substance to be useful as an effective pathologic marker, its quantity or quality is desired to change distinctly in correspondence with the pathema. In reality, however, physiologically active substances are found also in normal cells, or qualitatively hardly distinguishable form those derived from normal cells in many cases. Especially, with respect to a cancer marker, no marker has yet been discovered which is qualitatively and quantatively effective.

Under these circumstances, the present inventors have conducted extensive researches on the intracellular behavior of cathepsin E and as a result, have surprisingly found that very many abnormal cells exist which secrete proteins reactive with anti-cathepsin E antibodies, out of the cells. For example, such proteins are secreted from various cancer cells such as stomach cancer cells or pancreatic cancer cells. Further surprisingly, it has been found that despite their reactivity with anti-cathepsin E antibodies, their molecular weights are different from the molecular weight of usual cathepsin E.

Namely, the present invention provides a protein secreted by cancer cells, which has the following characteristics:
a) it is reactive with an anti-cathepsin E antibody,
b) its molecular weight is about 60,000 in the form of a monomer and about 120,000 in the form of a dimer,
c) it is secreted from pancreatic cancer cells, and
d) it has a protease activity.

Now, the present invention will be described in detail.

In the accompanying drawings;

Figure 1 is a graph showing the elution pattern of the pepstatin sephalose 4B chromatography in Example 2.

Figure 2 is a view showing the results of western blotting after SDS-polyacrylamide gel electrophoresis in Example 3.

Figure 3 is a view showing the results of measurement of the protease activity after the polyacrylamide gel electrophoresis in Example 4.

Conventional cathepsin E is an intracellular protease, and it is a surprising fact that the protein of the present invention is secreted out of cells specifically from abnormal cells such as cancer cells. The cells which secreted the protein of the present invention are abnormal cells which are not particularly limited and which include, for example, various cancer cells. Among them, stomach cancer cells and pancreatic cancer cells may particularly be mentioned. The protein of this invention is bound by a pepstatin column and is capable of decomposing hemoglobin even under an acidic condition. Therefore, it is considered to belong to a class of acidic proteases.

The protein of the present invention is a protein reactive with an antibody to conventional cathepsin E i.e. an anti-cathepsin E antibody. However, it is conceivable that such a protein is different from conventional cathepsin E in the specific activity, the molecular weight or the carbohydrate chain.

In fact, such a protein of the present invention secreted from cancer cells, is different in the molecular weight from cathepsin E present in normal cells. The molecular weight of the protein of the present invention secreted from pancreatic cancer cells is about 60,000 in the form of a monomer or about 120,000 in the form of a dimer and thus is distinctly different from the molecular weight of cathepsin E (80,000 in the form a dimer) present in normal cells. This indicates that a specific modification (to e.g. the carbohydrate chain) has been done by the pancreatic cancer cells, or molecular species specific to pancreatic cancer have been produced.

There is no particular restriction as to the method for culturing cells which secrete the protein of the present invention. Cancer cells such as stomach cancer cells or pancreatic cancer cells may be cultured in accordance with a common culturing method for animal cells.

Purification from the culture supernatant of the protein of the present invention secreted form such cells is not particularly limited and may be conducted in the same manner as a conventional method for purifying proteins. Among conventional purification methods for proteins, a pepstatin affinity chromatography using pepstatin is a preferred purification method, since it is thereby possible to remarkably improve the purification rate of the protein of the present invention.

The qualitative and quantitative analyses of the protein of the present invention secreted from the cells may be conducted usually by well known biochemical methods. For example, the protein can be measured immunologically, and a sandwich EIA method, a competition method or an agglutination reaction method may, for example, be mentioned. For example, in the sandwich EIA method, an antibody which is capable of recognizing the protein of the present invention and a sample are used to form an immunological complex of antibody-object-antibody. Such a complex is detected, and the obtained results are compared with e.g. a calibration curve obtained by measuring samples of known concentrations to determine the concentration in the sample of the protein of the present invention. In such an immunoassay, it is advisable to employ a label such as enzyme, a radioactive substance, a fluorescent substance, a chemoluminescent substance or a bioluminescent substance to facilitate the detection.

The antibody which is capable of recognizing the protein of the present invention, may be prepared by a conventional method using the protein of the present invention as the immunogen. Further, the animal to be immunized with the antigen may be an animal which is commonly used and is not particularly limited. For example, a goat, a sheep, a rabbit or a rat may be mentioned. Further, a polyclonal antibody or a monoclonal antibody prepared by a conventional method, may be used. The protein of the present invention has a reactivity with an anti-cathepsin E antibody, and such an antibody may also be employed.

The protein of the present invention is a novel protease which is different from conventional cathepsin E, although it has a reactivity with an anti-cathepsin E antibody. Such a protein of the present invention is secreted out of cells from abnormal cells such as cancer cells, whereby it is expected that early diagnosis of various diseases can be made possible by measuring the amount of the protein in the serum or body fluid such as pancreatic juice or gastric juice without sampling the target tissue. Further, it is conceivable to utilize the protein of the present invention as a marker specific to each particular cancer, and it will be possible to determine the diseased site by establishing a measuring method specific to the protein of the present invention secreted from the respective cells.

Further, from the fact that the protein of the present invention is secreted from abnormal cells represented by cancer cells, it is expected that the mechanism of canceration can be made clear, and a cancer drug may be developed by investigating the relation between canceration of cells and secretion of cathepsin E.

Now, the present invention will be described in further detail with reference to Examples. However, it should be understood that the present invention is by no means restricted to such specific Examples.

### Example 1

### Culturing of pancreatic cells

Using F-12 Nutrient Mixture (Ham) culture medium manufactured by GIBCO Co., pancreatic cancer cells of human origin were cultured by a usual method, whereupon 1.0 ℓ in total of the culture supernatant was collected. The collected culture supernatant was freeze-dried.

### EXAMPLE 2

### Purification of the culture supernatant by a column

The freeze-dried product obtained in Example 1 was dissolved in a 0.1 M sodium acetate buffer solution (pH 3.5) containing 1.0 M sodium chloride, and insoluble matters were removed by centrifugal separation to obtain a solution, and 6.0 ml of such a solution was applied to a pepstatin sephalose-4B column (1.0 x 15 cm) equilibrated with the same buffer solution. Then, the column was washed with the same solvent until absorption (280 nm) by the protein was no longer detected in the eluate from the column. Then, the protein was eluated from fraction No. 20 with a 0.1M Tris-HCl (pH 8.6) containing 1.0M sodium chloride.

The enzyme activity was measured under following conditions. Namely, 80 µl of the enzyme solution was added to 400 µl of a 2% hemoglobin solution (0.2M glycinehydrochloric acid buffer solution, pH 3.0), and the mixture was reacted at 37°C for a predetermined period. Then, 2.0 ml of 5% TCA cooled with ice, was added thereto, and the mixture was left to stand for at least 15 minutes in ice. Then, it was subjected to centrifugal separation for 10 minutes with 1500 x g, and the absorbance at 280 nm of the supernatant was measured to determine the activity. Calculation was conducted by 1U = 1ABS/min.

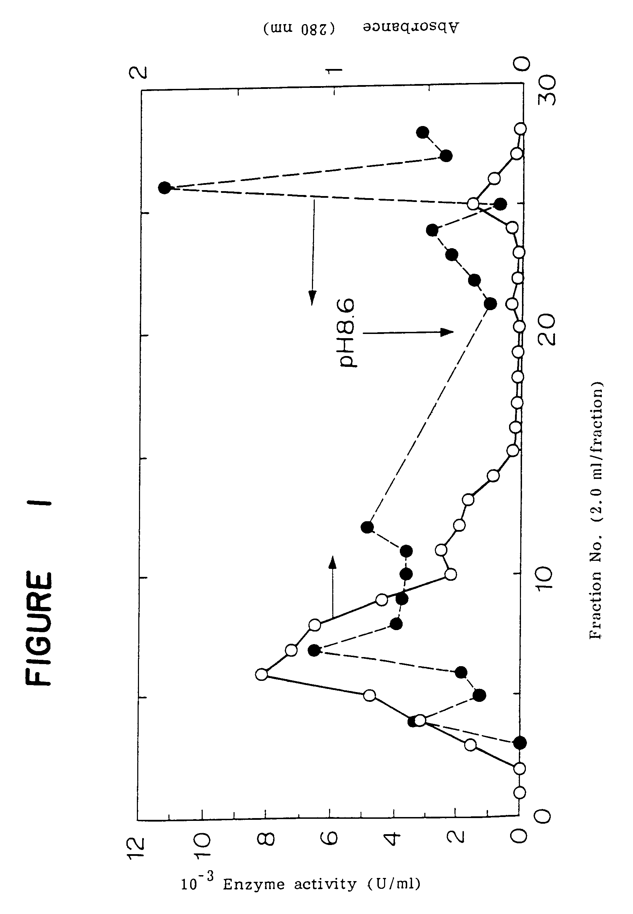

The results are shown in Figure 1.

In Figure 1, the abscissa represents the fraction No., the ordinate on the right represents the enzyme activity, and the ordinate on the left represents the absorbance at 280nm. As is evident from this Figure, Fraction No. 26 having a high enzyme activity, was recovered.

### EXAMPLE 3

### Analysis of the purified protein

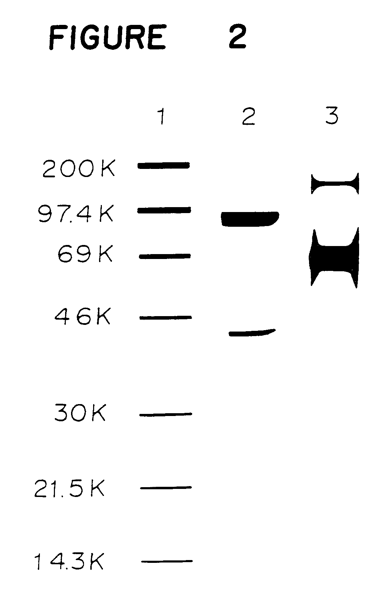

The protein solution recovered in Example 2 was developed by a 10% SDS-polyacryamide gel electrophoresis, and then the protein was transferred and adsorbed on a zeta-probe membrane (manufactured by BioRad company) by a western blotting method. An anti-human cathepsin E rabbit serum and the above zeta-probe membrane were contacted. Then, the membrane was washed, and an anti-rabbit IgG antibody bonded to biotin, was added thereto. The membrane was washed, and horse radish peroxitase-bonded avidin was added thereto. The membrane was washed and then treated with western blotting detection reagent ECL (RPN 2106) manufactured by Amersham Co. to develop luminescence at the portion where the horse radish peroxidaze was present. Then, the membrane and Hyper film-ECL manufactured by Amersham Co. were contacted, and the film was exposed and then developed. The results are shown in Figure 2.

In Figure 2, Lane No. 1, Lane No. 2 and Lane No. 3 represent the results obtained by developing under the same conditions the molecular weight marker, the conventional cathepsin E and the protein solution obtained in Example 2, respectively, and the numerical values indicate kDa. As a result, it was found that the protein recovered in Example 2 was reactive with the anti-human cathepsin E rabbit serum. It was further found that the molecular weight of the protein was different from the molecular weight of the conventional cathepsin E and was about 60,000 in the form of a monomer and about 120,000 in the form of a dimer. Further, as compared with the conventional cathepsin E, the band was not so sharp, which indicate a possibility that hyperglycosylation took place.

### EXAMPLE 4

### Analysis of purified cathepsin E

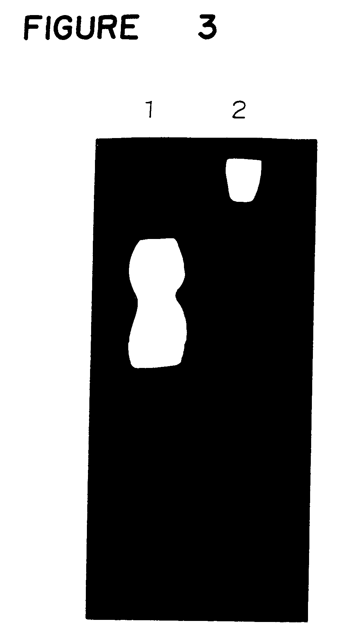

The protein solution recovered in Example 2, was developed by a 8% polyacrylamide gel electrophoresis. Then, the gel after the electrophoresis was immersed in a 0.67% hemoglobin solution under an acidic condition of 0.1N hydrochloric acid and incubated for 20 minutes. The hemoglobin solution was removed, and then incubation was continued for further 30 minutes in a 0.1N hydrochloric acid solution. Then, the gel was stained with a CBB solution and then depigmented. The results are shown in Figure 3, Lane No. 1 and Lane No. 2 shown the results obtained by developing the conventional cathepsin E and the protein solution obtained in Example 2, respectively.

It is evident from the Figure that the protein recovered in Example 2 exhibits a protease activity like the conventional cathepsin E.

## Claims

1. A protein secreted by cancer cells, which has the following characteristics:
a) it is reactive with an anti-cathepsin E antibody,
b) its molecular weight is about 60,000 in the form of a monomer and about 120,000 in the form of a dimer,
c) it is secreted from pancreatic cancer cells, and
d) it has a protease activity.

2. A method for diagnosing cancer, which comprises reacting an antibody to cathepsin E with a sample and detecting a complex of the antibody with the protein as defined in Claim 1 in the sample, the presence of such complex being indicative of the presence of cancer.

3. A method for diagnosing cancer, which comprises reacting the protein as defined in Claim 1 as an antigen with a sample and detecting a complex of the antigen with an antibody to the protein as defined in Claim 1 in the sample, the presence of such complex being indicative of the presence of cancer.

4. A method for diagnosing cancer, which comprises reacting DNA or RNA capable of hybridizing with mRNA of cathepsin E, with a sample and detecting a hybridization product of DNA or RNA with mRNA of the protein as defined in Claim 1 in the sample the presence of such hybridization product being indicative of the presence of cancer.
